(19)

Europäisches
Patentamt
European
Patent Office
Office européen
des brevets

(11)     **EP 3 064 195 A1**

(12)     **EUROPEAN PATENT APPLICATION**

(43) Date of publication:
07.09.2016   Bulletin 2016/36

(51) Int Cl.:
*A61K 8/19* (2006.01)          *A61Q 3/02* (2006.01)
*A61K 8/04* (2006.01)

(21) Application number: 16155266.6

(22) Date of filing: 11.02.2016

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority:   06.03.2015   US 201514640572

(71) Applicant: **Mina Atramentum, LLC**
**High Falls, NY 12440 (US)**

(72) Inventor: **Vancza,, Veleta**
**High Falls, NY New York 12440 (US)**

(74) Representative: **Adamson Jones**
**BioCity Nottingham**
**Pennyfoot Street**
**Nottingham NG1 1GF (GB)**

(54)     **COMPOSITION HAVING EARTH MATERIALS COMPRISING A PIGMENT**

(57)     A nail polish composition including a suspension base and a pigment, wherein at least 80% of the pigment is comprised of a noble metal, is provided. Furthermore, an associated method is also provided.

EP 3 064 195 A1

**Description**

CROSS-REFERENCE TO RELATED APPLICATIONS

This application claims priority to and the benefit of U.S. Provisional Application No. 61/949,575, filed March 7, 2014, and entitled "Composition Having Earth Materials Comprising the Pigment."

FIELD OF TECHNOLOGY

The following relates to a composition that utilizes earth materials as the pigment.

BACKGROUND

Nail lacquers or nail polishes can be purchased in a wide variety of colors. Traditionally, the color of the nail polish is created by using various pigments. Those pigments are typically synthetic materials. Moreover, traditional nail lacquers include toxic ingredients, such as formaldehyde, dibutyl phthalate (DBP), toluene, formaldehyde resin, and camphor.
Thus, a need exists for an apparatus and method for a nail lacquer composition that utilizes earth materials as the pigment, while also being free of common toxic ingredients.

SUMMARY

A first aspect relates generally to a composition comprising: a suspension base; and a pigment, wherein at least 80% of the pigment is comprised of a noble metal.
A second aspect relates generally to a composition comprising a suspension base; and a pigment for coloring the composition, the pigment having a weight/volume percentage with respect to a total volume of the composition above 5%, wherein the pigment comprises at least one of a noble metal and a gemstone.
A third aspect relates generally to a method of making a nail polish comprising adding a pigment to a suspension medium to form the nail polish, wherein at least 80% of the pigment is comprised of a noble metal.
The foregoing and other features of construction and operation will be more readily understood and fully appreciated from the following detailed disclosure, taken in conjunction with accompanying drawings.

DETAILED DESCRIPTION

A detailed description of the hereinafter described embodiments of the disclosed apparatus and method are presented herein by way of exemplification and not limitation with reference to the Examples provided herein. Although certain embodiments are shown and described in detail, it should be understood that various changes and modifications may be made without departing from the scope of the appended claims. The scope of the present disclosure will in no way be limited to the number of constituting components, the materials thereof, the shapes thereof, the relative arrangement thereof, etc., and are disclosed simply as an example of embodiments of the present disclosure.
As a preface to the detailed description, it should be noted that, as used in this specification and the appended claims, the singular forms "a", "an" and "the" include plural referents, unless the context clearly dictates otherwise.
Embodiments of the composition having earth materials, such as natural precious materials, forming the pigment may be a nail polish or nail lacquer that can that can be topically applied to fingernails or toe nails. Embodiments of the composition may also be applied to skin or other surfaces, inorganic or organic.
Embodiments of the composition may include a suspension medium and a pigment, wherein at least 80% of the pigment is comprised of a noble metal.
Embodiments of the composition may include a suspension medium. Embodiments of the suspension medium may be a suspension base, a base solution, a base nail polish, a base nail lacquer, a nail polish suspension base, a nail lacquer suspension base, a gel base, a gel polish, a high gloss base, a holographic base, a lacquer, a base coat, a top coat, a matte base, or any solution or suspension medium that can be combined with a pigment to form a nail polish composition. Further embodiments of the suspension medium may be clear or translucent. Embodiments of the suspension medium may be comprised of nitrocellulose. The suspension medium may also include various additives and fillers that are known to those having skill in the art. In an exemplary embodiment, the suspension medium may be may be 5-Free, meaning it may be free of 5 toxic ingredients found in many nail polishes -- formaldehyde, dibutyl phthalate (DBP), toluene, formaldehyde resin and camphor.
Embodiments of the composition may also include a pigment. The pigment may be a material or a combination of materials used for coloring the composition. The composition may include one or more pigments. Embodiments of the pigments used in the composition may be metals, precious and semiprecious gemstones, precious metals, noble metals, and the like. For example, embodiments of the pigment that are precious metals are gold, platinum, and silver. Other materials that may be pigments include tin, aluminum, graphite, diamond, sapphire, emerald, turquoise, tourmaline, spinel, sugilite, citrine, lapis lazuli, quartz, iolite, nephrite, ruby, amethyst, peach jasper, garnet, red jasper, red carnelian, opal, topaz, aquamarine, peridot, hematite, tiger eye, sodalite, amazonite, and the like. Moreover, a percentage of the pigment of the composition may be made of only precious metals. In one embodiment, the composition may comprise a pigment, wherein the pigment is comprised of 100% of precious metals. In another embodiment, a percentage of the pigment is comprised of at least 80% precious metals. In yet another embodiment, the percentage of pigment that is formed by precious metals is between 80% and 100%. In other embodiments, the percentage of pigment comprised from precious metal is below 80%.

Furthermore, embodiments of the pigment comprising one or more precious metals may have an effective particle size. The size of the particle of the precious metal may affect the consistency of the nail lacquer. The desired particle size of the precious metal used as the pigment may depend on the precious metal. For instance, an effective particle size of 10-24K gold used as a pigment ranges between 3-10$\mu$, but may also have an effective particle size as large as 80$\mu$. An effective particle size of platinum used as a pigment ranges between 3-10$\mu$, but may also have an effective particle size as large as 80$\mu$. An effective particle size of silver used as a pigment ranges between 3-10$\mu$, but may also have an effective particle size as large as 80$\mu$. Other metals that may be used in combination with the precious metals of gold, silver, and platinum, such as aluminum, tin, and graphite may each also have an affective particle size. For instance, an effective particle size of aluminum used a pigment or as a pigment combination with a precious metal(s) ranges between 60-70$\mu$, but may also have an effective particle size as large as 75$\mu$. An effective particle size of tin used a pigment or as a pigment combination with a precious metal(s) ranges between 25-45$\mu$, but may also have an effective particle size as large as 75$\mu$. An effective particle size of graphite used a pigment or as a pigment combination with a precious metal(s) ranges between 6-10$\mu$, but may also have an effective particle size as large as 20$\mu$. Gemstones that may be used as a pigment or a combination with one or more precious metals may have an affective particle size that ranges between 3-25$\mu$, but may also have an effective particle size as large as 80$\mu$.

Embodiments of the composition may be described by the following examples. The following formulas have been applied in the following examples:

$$\frac{Weight\ of\ Pigment(g)}{Total\ Vol\ (mL)}\ x\ 100,\ \text{which calculates the wt/vol\%; and}$$

$$\frac{Mass\ of\ Precious\ Metal(g)}{Total\ Mass\ of\ Pigment\ (g)}\ x\ 100,\ \text{which calculates the \% of pigment comprised of precious metal.}$$

EXAMPLE 1

The following composition is an embodiment of a composition using a suspension medium and a pigment only of 24K gold, having a particle size between 3-10$\mu$:

| Weight of Precious Metal Pigment | Total Volume | Weight of Other Pigment | Wt/Vol % | % Pigment Made From Precious Metal |
|---|---|---|---|---|
| 2.5 g | 10mL | N/A | 25% | 100% |

EXAMPLE 2

The following composition is an embodiment of a composition using a suspension medium and a pigment only of 22K gold, having a particle size between 3-10$\mu$:

| Weight of Precious Metal Pigment | Total Volume | Weight of Other Pigment | Wt/Vol % | % Pigment Made From Precious Metal |
|---|---|---|---|---|
| 2 g | 10mL | N/A | 20% | 100% |

EXAMPLE 3

The following composition is an embodiment of a composition using a suspension medium and a pigment only of gold, having a particle size between 3-10$\mu$:

| Weight of Precious Metal Pigment | Total Volume | Weight of Other Pigment | Wt/Vol % | % Pigment Made From Precious Metal |
|---|---|---|---|---|
| 0.6 g | 10mL | N/A | 6% | 100% |

EXAMPLE 4

The following composition is an embodiment of a composition using a suspension medium and a pigment being made of a combination of gold having a particle size between 3-10μ and graphite, having a particle size between 6-10μ:

| Weight of Precious Metal Pigment | Total Volume | Weight of Other Pigment | Wt/Vol % | % Pigment Made From Precious Metal |
|---|---|---|---|---|
| 0.6 g | 10mL | 0.8g | 6%: 8% | 42.8% |

EXAMPLE 5

The following composition is an embodiment of a composition using a suspension medium and a pigment being made of platinum having a particle size between 70-75μ:

| Weight of Precious Metal Pigment | Total Volume | Weight of Other Pigment | Wt/Vol % | % Pigment Made From Precious Metal |
|---|---|---|---|---|
| 2.5 g | 10mL | N/A | 25% | 100% |

EXAMPLE 6

The following composition is an embodiment of a composition using a suspension medium and a pigment being made of silver having a particle size between 3-10μ:

| Weight of Precious Metal Pigment | Total Volume | Weight of Other Pigment | Wt/Vol % | % Pigment Made From Precious Metal |
|---|---|---|---|---|
| 3 g | 10mL | N/A | 30% | 100% |

EXAMPLE 7

The following composition is an embodiment of a composition using a suspension medium and a pigment being made of silver having a particle size between 40-45μ:

| Weight of Precious Metal Pigment | Total Volume | Weight of Other Pigment | Wt/Vol % | % Pigment Made From Precious Metal |
|---|---|---|---|---|
| 0.32 g | 10mL | N/A | 3.2% | 100% |

EXAMPLE 8

The following composition is an embodiment of a composition using a suspension medium and a pigment being made of a combination of gold 24K having a particle size between 3-10μ and a synthetic pigment:

| Weight of Precious Metal Pigment | Total Volume | Weight of Other Pigment | Wt/Vol % | % Pigment Made From Precious Metal |
|---|---|---|---|---|
| 2.5 g | 10ml | 0.625 g | 25% : 6.25% | 80% |

EXAMPLE 9

The following composition is an embodiment of a composition using a suspension medium and a pigment being made of gold 24K having a particle size between 3-10μ and a synthetic pigment:

| Weight of Precious Metal Pigment | Total Volume | Weight of Other Pigment | Wt/Vol % | % Pigment Made From Precious Metal |
|---|---|---|---|---|
| 3 g | 10mL | 0.5g | 30% : 5% | 85.7% |

EXAMPLE 10

The following composition is an embodiment of a composition using a suspension medium and a pigment being made of a combination of silver having a particle size between 3-10μ and an synthetic pigment:

| Weight of Precious Metal Pigment | Total Volume | Weight of Other Pigment | Wt/Vol % | % Pigment Made From Precious Metal |
|---|---|---|---|---|
| 3.5 g | 10mL | 0.2g | 20% | 94.6% |

Further examples include compositions that include other metals as the pigment, along with a suspension medium, wherein at least 80% of the pigment is comprised of metals and/or gemstones. For example, a composition including tin as the pigment may include 6.4g in 10mL, or 64% (wt/vol%). An embodiment of a composition that includes aluminum as the pigment may include 0.75g in 10mL, or 7.5% (wt/vol%). An embodiment of a composition that includes graphite as the pigment may include 0.8g in 10mL, or 8% (wt/vol%). An embodiment of a composition that includes a gemstone as the pigment may include 1g in 10mL, or 10% (wt/vol%).

Even further embodiments of a composition utilizing a precious metal as a pigment may include paints, such as automotive paints. In this embodiment, the suspension medium may be a waterborne or solventborne binder, as used in automotive basecoats. A precious metal may be added to the basecoat to form a composition that may be pneumatically sprayed onto automobiles or other vehicles or any surface that may require a basecoat of paint. Further, in this embodiment, a precious metal, such as gold 24K may a weight/volume percentage between 25-45% (e.g. 2.5-4.5g in 10mL binder), wherein a precious metal comprises 80% to a 100% of the pigment that forms the color of the paint.

The examples provided herein document a percentage of pigment comprised of a precious metal, but it is understood that the some suspension mediums that may be used in various embodiments of the composition may affect or comprise at least a portion of the pigment. Thus, the percentage pigment may only account for materials in the composition that are configured to or intended to cause coloration. In addition, the suspension medium may be separate elements forming the claimed composition.

While this disclosure has been described in conjunction with the specific embodiments outlined above, it is evident that many alternatives, modifications and variations will be apparent to those skilled in the art. Accordingly, the preferred embodiments of the present disclosure as set forth above are intended to be illustrative, not limiting. Various changes may be made without departing from the spirit and scope of the invention, as required by the following claims. The claims provide the scope of the coverage of the invention and should not be limited to the specific examples provided herein.

**Claims**

1. A composition comprising:

   a suspension base; and
   a pigment, wherein at least 80% of the pigment is comprised of a noble metal.

2. The composition of claim 1, wherein the noble metal has a particle size of 3-80μ.

3. The composition of claim 1, wherein the noble metal is at least one of gold, silver, and platinum.

4. The composition of claim 1, wherein the suspension base is a clear or translucent nail polish solution.

**5.** The composition of claim 1, wherein the suspension medium is a binder for making a basecoat of automotive paint.

**6.** A composition comprising:

a suspension base; and
a pigment for coloring the composition, the pigment having a weight/volume percentage with respect to a total volume of the composition above 5%;
wherein the pigment comprises at least one of a noble metal and a gemstone.

**7.** The composition of claim 6, wherein the pigment has a particle size of 3-80$\mu$.

**8.** The composition of claim 6, wherein the noble metal includes at least one of gold, silver, and platinum.

**9.** The composition of claim 6, wherein the suspension base is a clear or translucent nail polish solution.

**10.** The composition of claim 6, wherein the suspension medium is a binder for making a basecoat of automotive paint.

**11.** A method of making a nail polish comprising:

adding a pigment to a suspension medium to form the nail polish, wherein at least 80% of the pigment is comprised of a noble metal.

**12.** The method of claim 11, wherein the noble metal has a particle size of 3-80$\mu$.

**13.** The method of claim 11, wherein the noble metal is at least one of gold, silver, and platinum.

**14.** The method of claim 11, wherein the suspension base is a clear or translucent nail polish solution.

Europäisches
Patentamt

European
Patent Office

Office européen
des brevets

## EUROPEAN SEARCH REPORT

Application Number

EP 16 15 5266

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 3 713 870 A (KAYE M) 30 January 1973 (1973-01-30) * column 3; example 3 * ----- | 1-14 | INV. A61K8/19 A61Q3/02 A61K8/04 |
| X | EP 1 413 284 A1 (NIPPON SHEET GLASS CO LTD [JP]) 28 April 2004 (2004-04-28) * page 6; examples 1-6, 7 * * page 8, paragraph 51 - page 9, paragraph 55 * * claims 1-3 * * page 2, paragraphs 1, 5-9 * ----- | 1-14 | |
| X | FR 2 959 665 A1 (BLANC JACQUES [FR]) 11 November 2011 (2011-11-11) * claims * ----- | 1-14 | |
| X | EP 2 348 076 A1 (NIPPON SHEET GLASS CO LTD [JP]) 27 July 2011 (2011-07-27) * page 17, paragraph 151 - page 18, paragraph 156 * * page 6, paragraph 39 * * page 7, paragraph 41 * ----- | 1-5, 10-14 | |
| X | EP 2 147 954 A1 (NIPPON SHEET GLASS CO LTD [JP]) 27 January 2010 (2010-01-27) * claims * * page 17, paragraph 170 * ----- | 1-14 | TECHNICAL FIELDS SEARCHED (IPC) A61K A61Q |
| X | US 2003/077238 A1 (ROOVERS MARIA MAGDALENA CATHAR [NL] ET AL) 24 April 2003 (2003-04-24) * claims 3-12, 22-27 * * paragraph [0098] * * paragraph [0124]; claim 11 * ----- | 1-14 | |

-/--

The present search report has been drawn up for all claims

| Place of search | Date of completion of the search | Examiner |
|---|---|---|
| Munich | 9 June 2016 | Lemarchand, Aude |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                         
& : member of the same patent family, corresponding document

EPO FORM 1503 03.82 (P04C01)

2

Europäisches
Patentamt

European
Patent Office

Office européen
des brevets

# EUROPEAN SEARCH REPORT

Application Number

EP 16 15 5266

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 99/36478 A1 (MINNESOTA MINING & MFG [US]; WHITNEY LELAND R [US]; OUDERKIRK ANDREW J) 22 July 1999 (1999-07-22)<br>* claims 38-40 *<br>* page 1, lines 5-15, 28-31 *<br>* page 4, lines 1-2 * | 1-5, 11-14 | |
| X,P | US 2015/328090 A1 (VANCZA VELETA [US]) 19 November 2015 (2015-11-19)<br>* claims * | 1-14 | |
| X | Anonymous: "GNPD - 18K White Gold and Silver Top Coat",<br>ID 2261496,<br>1 December 2013 (2013-12-01), XP055279455,<br>Retrieved from the Internet:<br>URL:http://www.gnpd.com/sinatra/recordpage/2261496/from_search/MnYlHH9DAQ/?page=1<br>[retrieved on 2016-06-09]<br>* the whole document * | 1-14 | |

TECHNICAL FIELDS
SEARCHED        (IPC)

The present search report has been drawn up for all claims

| Place of search | Date of completion of the search | Examiner |
|---|---|---|
| Munich | 9 June 2016 | Lemarchand, Aude |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 16 15 5266

This annex lists the patent family members relating to the patent documents cited in the above-mentioned European search report.
The members are as contained in the European Patent Office EDP file on
The European Patent Office is in no way liable for these particulars which are merely given for the purpose of information.

09-06-2016

| Patent document cited in search report | | Publication date | Patent family member(s) | | Publication date |
|---|---|---|---|---|---|
| US 3713870 | A | 30-01-1973 | NONE | | |
| EP 1413284 | A1 | 28-04-2004 | BR | 0205864 A | 21-10-2003 |
| | | | CN | 1476319 A | 18-02-2004 |
| | | | EP | 1413284 A1 | 28-04-2004 |
| | | | KR | 20040021578 A | 10-03-2004 |
| | | | US | 2004213820 A1 | 28-10-2004 |
| | | | WO | 03011235 A1 | 13-02-2003 |
| FR 2959665 | A1 | 11-11-2011 | NONE | | |
| EP 2348076 | A1 | 27-07-2011 | EP | 2348076 A1 | 27-07-2011 |
| | | | WO | 2010024256 A1 | 04-03-2010 |
| EP 2147954 | A1 | 27-01-2010 | CN | 101675120 A | 17-03-2010 |
| | | | EP | 2147954 A1 | 27-01-2010 |
| | | | JP | 5154547 B2 | 27-02-2013 |
| | | | US | 2010129412 A1 | 27-05-2010 |
| | | | WO | 2008130040 A1 | 30-10-2008 |
| US 2003077238 | A1 | 24-04-2003 | AU | 2002324895 A1 | 24-03-2003 |
| | | | BR | 0205973 A | 30-12-2003 |
| | | | US | 2003077238 A1 | 24-04-2003 |
| | | | WO | 03022206 A2 | 20-03-2003 |
| WO 9936478 | A1 | 22-07-1999 | BR | 9906908 A | 10-10-2000 |
| | | | DE | 69902876 D1 | 17-10-2002 |
| | | | DE | 69902876 T2 | 15-05-2003 |
| | | | EP | 1047732 A1 | 02-11-2000 |
| | | | JP | 2002509044 A | 26-03-2002 |
| | | | US | 6475609 B1 | 05-11-2002 |
| | | | WO | 9936478 A1 | 22-07-1999 |
| US 2015328090 | A1 | 19-11-2015 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61949575 A